# EUROPÄISCHE PATENTSCHRIFT

(11) **EP 3 229 912 B1**
(45) Veröffentlichungstag und Bekanntmachung des Hinweises auf die Patenterteilung: **08.07.2020**
(21) Anmeldenummer: 15787624.4
(22) Anmeldetag: 02.11.2015
(51) Int. Cl.: A61Q 5/06, A61K 8/73, A61K 8/81

(54) **MITTEL UND VERFAHREN ZUR TEMPORÄREN VERFORMUNG KERATINHALTIGER FASERN**
COMPOSITIONS AND METHODS FOR TEMPORARILY SHAPING KERATIN-CONTAINING FIBERS
COMPOSITIONS ET METHODES POUR DEFORMER TEMPORAIREMENT DES FIBRES KERATINIQUES

(30) Priorität: 10.12.2014 DE 102014225442
(43) Veröffentlichungstag der Anmeldung: 18.10.2017
(73) Patentinhaber: Henkel AG & Co. KGaA, 40589 Düsseldorf (DE)
(72) Erfinder: KNAPPE, Thorsten, 22869 Schenefeld (DE); FÖRSTER, Thomas, 40591 Düsseldorf (DE); BAYERSDÖRFER, Rolf, 22589 Hamburg (DE)
(86) Internationale Anmeldenummer: PCT/EP2015/075378
(87) Internationale Veröffentlichungsnummer: WO 2016/091465

(56) Entgegenhaltungen:
- WO-A1-99/66888
- WO-A2-2011/076490
- US-A- 3 372 840
- US-A1- 2002 074 349
- US-A1- 2009 098 079
- US-A1- 2013 018 333

## Beschreibung

Die Anmeldung betrifft das technische Fachgebiet der temporären Umformung keratinhaltiger Fasern, insbesondere menschlicher Haare. Gegenstand der Anmeldung sind spezifische haarkosmetische Formulierungen, welche sich zur Applikation auf keratinhaltige Fasern mittels eines Entspannungsverdampfungsverfahrens eignen. Darüber hinaus sind die Verwendung dieser haarkosmetischen Formulierungen in Vorrichtungen zur Entspannungsverdampfung und Verfahren zur temporären Umformung keratinhaltiger Fasern Gegenstand der vorliegenden Anmeldung Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, welche sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, welche einer permanenten oder temporären Formgebung der Haare dienen, eine wichtige Rolle. Während bei der permanenten Umformung die chemische Struktur der keratinhaltigen Faser durch Reduktion und Oxidation modifiziert wird, finden solche Modifikationen der chemischen Struktur bei der temporären Umformung nicht statt. Entsprechende Mittel zur temporären Verformung enthalten als festigenden Wirkstoff üblicherweise synthetische Polymere und/oder Wachse.

Die wichtigste Eigenschaft eines Mittels zur temporären Verformung keratinhaltiger Fasern, im Folgenden auch Stylingmittel genannt, besteht darin, den behandelten Fasern in der neu modellierten Form - d.h. einer den Fasern aufgeprägten Form - einen möglichst starken Halt zu geben. Handelt es sich bei den keratinhaltigen Fasern um menschliche Haare, spricht man auch von starkem Frisurenhalt oder von hohen Haltegrad des Stylingmittels. Der Frisurenhalt wird im Wesentlichen durch die Art und Menge der eingesetzten festigenden Wirkstoffe bestimmt, wobei jedoch auch ein Einfluss der weiteren Bestandteile des Stylingmittels sowie der Applikationsform gegeben sein kann.

Im Bereich der temporären Verformung keratinhaltiger Fasern hat insbesondere die Sprühapplikation entsprechender kosmetischer Zubereitungen eine große Bedeutung, wobei die Zubereitungen in der Regel als Pumpsprays oder Aerosolsprays appliziert werden (so wie zum Beispiel in der WO 2011/076490 beschrieben). Hierzu werden die kosmetischen Zubereitungen in einer Abgabevorrichtung konfektioniert, aus denen sie entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation haarkosmetischer Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann.

Vor diesem Hintergrund besteht ein Bedarf nach alternativen Wegen zur Zerstäubung haarkosmetischer Zubereitungen. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation haarkosmetischer Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel.

Aufgabe der vorliegenden Erfindung war es daher, spezifische haarkosmetische Zubereitungen zur temporären Verformung keratinhaltiger Fasern zur Verfügung zu stellen, welche sich aufgrund ihrer chemischen und physikalischen Eigenschaften zur zielgerichteten Sprühapplikation mittels einer Vorrichtung zur Entspannungsverdampfung eignen. Weiterhin sollen die Zubereitungen geeignet sein, nach einer Applikation mittels eines Entspannungsverfahrens einen hohen Haltegrad, insbesondere einen hohen Langzeithaltegrad, und einen hohen Volumeneffekt zu realisieren. Es hat sich gezeigt, dass zur Lösung dieser Aufgabe aus der Vielzahl bekannten haarkosmetisch wirksamen Polymerzubereitungen insbesondere lösungsmittelhaltige Zubereitungen auf Grundlage einer Kombination filmbildender Polymere mit Chitosan und dessen Derivaten geeignet sind.

Ein erster Gegenstand der vorliegenden Erfindung ist somit ein kosmetisches Produkt, umfassend
a) eine kosmetische Zubereitung, enthaltend bezogen auf ihr Gesamtgewicht
   a1) 45 bis 98 Gew.-% mindestens eines polaren Lösungsmittels;
   a2) 0,1 bis 4,0 Gew.-% Chitosan
   a3) 0,1 bis 15 Gew.-% filmbildendes Polymer
b) eine Vorrichtung zur Entspannungsverdampfung der kosmetischen Zubereitung a).

Dabei ist die Vorrichtung zur Entspannungsverdampfung im Sinne der vorliegenden Erfindung wie in den Ansprüchen beschrieben. Die kosmetische Zubereitung a) ist vorzugsweise flüssig. Die kosmetische Zubereitung a) kann als Lösung oder Dispersion, beispielsweise als Emulsion oder Suspension vorliegen. Bevorzugte kosmetische Zubereitungen a) liegen in Form einer Lösung oder einer Suspension vor.

Die erfindungsgemäße kosmetische Zubereitung enthält als ersten wesentlichen Bestandteil 45 bis 98 Gew.-% mindestens eines polaren Lösungsmittels a1). Bevorzugte kosmetische Produkte sind dadurch gekennzeichnet, dass der Gewichtsanteil des polaren Lösungsmittels a1) am Gesamtgewicht der kosmetischen Zubereitung a) 55 bis 95 Gew.-%, vorzugsweise 70 bis 92 Gew.-% beträgt. Entsprechende Mittel zeichnen sich durch eine gute kosmetische Wirkung bei gleichzeitig guter Applizierbarkeit aus.

Zur Verbesserung der Anwendungseigenschaften erfindungsgemäßer kosmetischer Zubereitungen bei gleichzeitiger Minimierung der thermischen Belastung etwaiger Wirk- oder Hilfsstoffe im Verlauf des Entspannungsverdampfungsverahrens, hat es sich als vorteilhaft erwiesen, polare Lösungsmittel a1) einzusetzen, welche eine Siedetemperatur (20°C, 1013 mbar) zwischen 50 und 110°C, vorzugsweise zwischen 70 und 105°C aufweisen. Als besonders geeignet haben sich dabei Ethanol, Isopropanol und Wasser erwiesen, welche aus diesem Grund als polare Lösungsmittel a1) bevorzugt werden.

Besonders bevorzugte polare Lösungsmittel a1) oder Lösungsmittelsysteme sind
- dadurch gekennzeichnet, dass der Gewichtsanteil von Wasser und Ethanol am Gesamtgewicht des polaren Lösungsmittels a1) mindestens 60 Gew.-%, bevorzugt mindestens 80 Gew.-%, besonders bevorzugt mindestens 90 Gew.-% und insbesondere mindestens 95 Gew.-% beträgt;
- dadurch gekennzeichnet, dass der Gewichtsanteil von Wasser am Gesamtgewicht des polaren Lösungsmittels a1) mehr als 80 Gew.-%, vorzugsweise mehr als 88 Gew.-% und insbesondere mehr als 92 Gew.-% beträgt.

Ganz besonders bevorzugte kosmetische Zubereitungen sind dadurch gekennzeichnet, dass der Anteil flüchtiger Bestandteile maximal 55 Gew.-% beträgt. Zur Gruppe dieser flüchtigen Bestandteile zählt u.a. auch das polare Lösungsmittel Ethanol. Ganz besonders bevorzugte kosmetische Zubereitungen sind demnach mit anderen Worten dadurch gekennzeichnet, dass der Gewichtsanteil des Ethanols am Gesamtgewicht der kosmetischen Zubereitung maximal 55 Gew.-%, vorzugsweise 10 bis 55 Gew.-%, besonders bevorzugt 25 bis 55 Gew.-% und insbesondere 40 bis 55 Gew.-% beträgt.

Ein zweiter wesentlicher Bestandteil erfindungsgemäßer kosmetischer Zusammensetzungen ist das Chitosan a2). In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil des Chitosans a2) am Gesamtgewicht der kosmetischen Zubereitung a) 0,1 bis 2,0 Gew.-%, vorzugsweise 0,2 bis 1,0 Gew.-% beträgt.

Chitosane stellen Biopolymere dar und werden zur Gruppe der Hydrokolloide gezählt. Chemisch betrachtet, handelt es sich um partiell deacetylierte Chitine unterschiedlichen Molekulargewichtes.

Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, die ein mittleres Molekulargewicht (Gewichtsmittel) von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen.

Neben den Chitosanen als typische Biopolymere kommen im Sinne der Erfindung auch kationisch derivatisierte Chitosane (wie z. B. Quaternierungsprodukte) oder alkoxylierte Chitosane als Derivate des Chitosans in Frage.

Erfindungsgemäß bevorzugte Mittel sind dadurch gekennzeichnet, dass sie als Chitosan mindestens ein Neutralisationsprodukt von Chitosan mit mindestens einer organischen Carbonsäure, wie insbesondere Ameisensäure, Essigsäure, Zitronensäure, Milchsäure, Pyrrolidoncarbonsäure, Weinsäure, Glycolsäure, Nicotinsäure, Hydroxyisobuttersäure, Hydroxyisovaleriansäure oder Gemischen dieser Säuren, enthalten. Hierbei ist es erfindungsgemäß bevorzugt, die organische Carbonsäure unter Milchsäure, Ameisensäure, Pyrrolidoncarbonsäure, Nicotinsäure, Hydroxyisobuttersäure, Hydroxyisovaleriansäure oder Gemischen dieser Säuren auszuwählen. Dieses Neutralisationsprodukt kann beispielsweise in einem wässrigen Medium durch Zugabe von Chitosan und der entsprechenden organischen Carbonsäure hergestellt werden.

Geeignete Chitosane sind beispielsweise unter den Handelsbezeichnungen Hydagen® CMF (1 Gew.-% Aktivsubstanz in wässriger Lösung mit 0.4 Gew.-% Glykolsäure, Molekulargewicht 500000 bis 5000000 g/mol, Cognis), Hydagen® HCMF (Chitosan zu 80 % deacetyliert, Molekulargewicht 50000 bis 1000000 g/mol, Cognis), Kytamer® PC (80 Gew.-% Aktivsubstanz an Chitosanpyrolidoncarboxylat (INCI-Bezeichnung: Chitosan PCA), Amerchol) und Chitolam® NB/101 im Handel frei verfügbar.

Ein dritter wesentlicher Bestandteil erfindungsgemäßer kosmetischer Zubereitungen a) sind die filmbildenden Polymere a3). Als filmbildende Polymere a3) eignen sich permanent als auch temporär kationische, anionische, nichtionische oder amphotere Polymere. Diese filmbildenden Polymere können synthetischen oder natürlichen Ursprungs sein. Bevorzugte kosmetische Zubereitung a) enthalten bezogen auf ihr Gesamtgewicht 1,0 bis 12 Gew.-%, vorzugsweise 2,0 bis 10 Gew.-% filmbildendes Polymer a3). Dieses Polymer a3) ist von dem Chitosan a2) verschieden.

Beispiele für gebräuchliche filmbildende Polymere a4) sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polyquaternium-68, Polyquaternium-69 Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Eine Gruppe mit Vorzug in der kosmetischen Zubereitung eingesetzten Polymere a3) sind die Vinylpyrrolidon Homo- oder Copolymere B. Mit besonderem Vorzug eingesetzte Polymere a3) sind beispielsweise:
- Polyvinylpyrrolidone, wie sie beispielsweise unter der Bezeichnung Luviskol® (BASF) vertrieben werden,
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind bevorzugte nichtionische Polymere.

Aufgrund ihrer kosmetischen Wirkung in Kombination mit dem Chitosan a2) erfindungsgemäß bevorzugt eingesetzte filmbildenden Polymere sind insbesondere die Polyvinylpyrrolidone (INCI-Bezeichnung: PVP) sowie die Vinylpyrrolidon/Vinylacetat-Copolymere (INCI-Bezeichnung VP/VA Copolymer). Bevorzugte kosmetisches Produkte sind dadurch gekennzeichnet, dass das filmbildende Polymer a3) ausgewählt ist aus der Gruppe der nichtionischen Polymere, vorzugsweise aus der Gruppe der Polyvinylpyrrolidone und VinylpyrrolidonNinylacetat-Copolymere, vorzugsweise der Vinylpyrrolidon/Vinylacetat-Copolymere.

Eine weiter Gruppe bevorzugter filmbildender Polymere a3) bilden die kationischen Polymere, insbesondere kationische Polymere mit den INCI Bezeichnungen Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polyquaternium-68 und Polyquaternium-69. Ganz besonders bevorzugte kosmetische Produkt sind dadurch gekennzeichnet, dass das filmbildende Polymer a3) ausgewählt ist aus der Gruppe der kationischen Polymere mit den INCI Bezeichnungen Polyquaternium-4, Polyquaternium-11, Polyquaternium-16, Polyquaternium-55, Polyquaternium-69. Entsprechende kosmetische Produkte zeichnen sich durch besonders gute haarkosmetische Eigenschaften, insbesondere Pflege- und Stylingeigenschaften aus.

Eine erste Gruppe besonders bevorzugter kationischer Polymere a3) sind die kationischen Cellulosederivate, insbesondere solche, die aus einer Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hervorgehen. Unter diesen kationischen Cellulosen sind wiederum solche kationischen Cellulosen mit der INCI-Bezeichnung Polyquaternium-4 besonders bevorzugt. Entsprechende Cellulosen werden beispielsweise unter den Bezeichnungen Celquat® H 100 und Celquat® L 200 von der Firma National Starch vertrieben.

Eine zweite Gruppe besonders bevorzugter kationischer Polymere a3) sind die Copolymere des Vinylpyrrolidons mit quaternierten Derivaten des Dialkylaminoalkylacrylats und -methacrylats, insbesondere mit Dietylsulfat quaternierte Copoylmere von Vinylpyrrolidon und Dimethylaminoethylmethacrylat (INCI-Bezeichnung: Polyquaternium-11). Entsprechende Polymere sind beispielsweise unter den Handelsbezeichnungen Gafquat® 440, Gafquat® 734 und Gafquat® 755 N (ISP) erhältlich.

Weitere bevorzugte kationsiche Polymere a3) sind N-Methylvinylimidazol/Vinylpyrrolidon-Copolymere (INCI-Bezeichnung: Polyquaternium-16), welche beispielsweise von der BASF unter den Handelsnamen Luviquat® Style, Luviquat® FC 370, Luviquat® FC 550, Luviquat® FC 905 und Luviquat® HM 552 vertrieben werden.

Bevorzugt sind weiterhin Copolymere aus Methacryloylaminopropyllauryldimethylammoniumchlorid mit Vinylpyrrolidon und Dimethylaminopropylmethacrylamid (INCI-Bezeichnung: Polyquaternium-55), die beispielsweise von der Firma ISP unter den Handelsnamen, Styleze® W-10 oder Styleze® W-20 erhältlich sind.

Ganz besonders bevorzugte kationische Polymere a3) sind schließlich Copolymere aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welche beispielsweise unter dem Handelsnamen AquaStyle® 300 (28-32 Gew.-% Aktivsubstanz in Ethanol-Wasser-Gemisch, Molekulargewicht 350000) von der Firma ISP vertrieben werden.

Aufgrund ihrer überdurchschnittlichen Stylingeigenschaften sind insbesondere solche kosmetischen Zubereitungen a) bevorzugt, die sowohl nichtionisches Polymer als auch kationisches Polymer a3) enthalten.

Die erfindungsgemäßen kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur Ti erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck p₀ < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, p₀ = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck po, zum Beispiel den umgebenden Luftdruck (po = 1,000 bar), entlastet werden kann, wodurch mindestens teilweise eine Verdampfung der kosmetischen Zubereitung a) erreicht wird.
Die kosmetische Zubereitung a) kann unmittelbar in dem Raum entspannt werden, in welchem sie zuvor erhitzt wurde. Die erhitzte und unter Überdruck befindliche kosmetische Zubereitung a) kann alternativ aber auch nach dem Erhitzen in einen zweiten Raum transportiert werden, in welchem dann nachfolgend die Druckentlastung erfolgt.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung kann die kosmetische Zubereitung a) einer Düse zugeführt werden, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher bevorzugt. Der spezifische Düsentyp oder die spezifische Düsengestaltung wird in Abhängigkeit von den jeweiligen Sprühnebeleigenschaften gezielt festgelegt.

Zusammenfassend verfügt eine bevorzugte Vorrichtung zur Entspannungsverdampfung über
b1) einen mittels eines Ventils zu verschließenden und zu öffnenden Behälter b1), welcher den geschlossenen Innenraum definiert, in dem die kosmetische Zubereitung aufgenommen werden kann,
b2) eine Heizvorrichtung b2), welche es ermöglicht eine in dem Behälter b1) befindliche kosmetische Zubereitung zu erhitzen.
Besonders bevorzugt ist der Einsatz einer zusätzlichen Düse b3), welche eine Zerstäubung der aus dem Behälter entweichenden kosmetischen Zubereitung a) ermöglicht. Alternativ zu einem Ventil kann auch ein vergleichbar wirkendes Schließelement, welches eine zugehörige Öffnung im Behälter durch entsprechende Positionsänderung verschließen oder freigeben kann, zum Einsatz kommen

Ein bevorzugter Gegenstand der vorliegenden Erfindung ist ein kosmetisches Produkt, umfassend
a) eine kosmetische Zubereitung, enthaltend bezogen auf ihr Gesamtgewicht
   a1) 45 bis 98 Gew.-% mindestens eines polaren Lösungsmittels;
   a2) 0,1 bis 4,0 Gew.-% Chitosan
   a3) 0,1 bis 15 Gew.-% filmbildendes Polymer;
b) eine Vorrichtung zur Entspannungsverdampfung der kosmetischen Zubereitung a), wobei die Vorrichtung zur Entspannungsverdampfung einen Behälter b1) und eine Heizvorrichtung b2 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   - die kosmetische Zubereitung a) in dem geschlossenen Innenraum des Behälters b1) mittels der Heizvorrichtung b2) unter Druckerhöhung erhitzt werden kann.

Ein besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 45 bis 98 Gew.-% mindestens eines polaren Lösungsmittels;
   a2) 0,1 bis 4,0 Gew.-% Chitosan
   a3) 0,1 bis 15 Gew.-% filmbildendes Polymer
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht ein in dem Behälter b1) befindliche kosmetische Zubereitung zu erhitzen
   b3) eine Düse b3), welche eine Zerstäubung der kosmetischen Zubereitung a) ermöglicht.

Ein weiterer bevorzugter Gegenstand der vorliegenden Erfindung ist daher ein kosmetisches Produkt,
umfassend
a) eine kosmetische Zubereitung, enthaltend bezogen auf ihr Gesamtgewicht
   a1) 45 bis 98 Gew.-% mindestens eines polaren Lösungsmittels, wobei der Gewichtsanteil von Wasser am Gesamtgewicht des polaren Lösungsmittels a1) vorzugsweise mindestens 80 Gew.-%, bevorzugt mindestens 88 Gew.-%, besonders bevorzugt mindestens 92 Gew.-% beträgt,
   a2) 0,1 bis 4,0 Gew.-% Chitosan
   a3) 0,1 bis 15 Gew.-% filmbildendes Polymer
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht ein in dem Behälter b1) befindliche kosmetische Zubereitung zu erhitzen
   b3) eine Düse b3), welche eine Zerstäubung der kosmetischen Zubereitung a) ermöglicht.

Ein besonders bevorzugter Gegenstand der vorliegenden Erfindung ist mit anderen Worten ein kosmetisches Produkt, umfassend
a) eine kosmetische Zubereitung, enthaltend bezogen auf ihr Gesamtgewicht
   a1) 45 bis 98 Gew.-% mindestens eines polaren Lösungsmittels;
   a2) 0,1 bis 4,0 Gew.-% Chitosan
   a3) 0,1 bis 15 Gew.-% filmbildendes Polymer;
b) eine Vorrichtung zur Entspannungsverdampfung der kosmetischen Zubereitung a), wobei die Vorrichtung zur Entspannungsverdampfung einen Behälter b1) und eine Heizvorrichtung b2 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   - die kosmetische Zubereitung a) in dem geschlossenen Innenraum des Behälters b1) mittels der Heizvorrichtung b2) unter Druckerhöhung erhitzt werden kann,
   - die erhitzte kosmetische Zubereitung a) aus dem Innenraum des Behälters b1) unter Druckminderung in die Umgebung entspannt werden kann.

Der Behälter b1), in welchem die kosmetische Zubereitung erhitzt wird, ist in einer Weise ausgestaltet, die es ermöglicht, diesen Behälter während der Erhitzung der kosmetischen Zubereitung a) gegen die Umgebung vollständig abzuschließen und nach der Erhitzung, zur Ermöglichung Entspannungsverdampfung der kosmetischen Zubereitung a), zu öffnen. Dies kann beispielsweise durch ein Bauteil zur Durchflussregelung, insbesondere ein Ventil, gewährleistet werden.

Der Behälter b1), in welchem die kosmetische Zubereitung erhitzt wird, steht vorzugsweise in Kontakt mit einem weiteren Behälter, aus welchem die zur Entspannungsverdampfung vorgesehene Menge der kosmetischen Zubereitung vor der Erhitzung in den Behälter b1) überführt wird. Der Zugang zwischen diesem Vorratsbehälter und Behälter b1) ist dabei über eine entsprechende Vorrichtung, beispielsweise ein Ventil, zu öffnen und zu schließen. Dieser weitere Behälter ist vorzugsweise in Form eines Vorratsbehälters ausgestaltet, das heißt, er umfasst bevorzugt ein Vielfaches, beispielsweise mehr als das Zehnfache, vorzugsweise mehr als das Fünfzigfache, der für einen Verdampfungsvorgang notwendigen Menge der kosmetischen Zubereitung. Mit anderen Worten weist der weitere Behälter/Vorratsbehälter vorzugsweise ein Vielfaches, beispielsweise mehr als das Zehnfache Volumen, vorzugsweise mehr als das Zwanzigfache und insbesondere mehr als das Fünfzigfache Volumen des Behälters b1) auf.

Ein weiterer besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 45 bis 98 Gew.-% mindestens eines polaren Lösungsmittels;
   a2) 0,1 bis 4,0 Gew.-% Chitosan
   a3) 0,1 bis 15 Gew.-% filmbildendes Polymer;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Zwanzigfache und insbesondere mindestens das Fünfzigfache Volumen des Behälters b1) aufweist.

Der Vorratsbehälter ist kein Druckbehälter und die in dem Vorratsbehälter befindliche kosmetische Zusammensetzung befindet sich nicht unter Druck, mit anderen Worten entspricht der Druck im Inneren des Vorratsbehälters dem Umgebungsdruck (auch Luftdruck oder Atmosphärendruck). So umfassen entsprechende kosmetische Produkte beispielsweise keine Treibmittel. Auch verfügt das kosmetische Produkt über keine Pumpvorrichtung, die geeignet ist, die kosmetische Zubereitung ohne die Einwirkung der Vorrichtung zur Entspannungsverdampfung in die Umgebung freizusetzen oder zu versprühen.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 45 bis 98 Gew.-% mindestens eines polaren Lösungsmittels;
   a2) 0,1 bis 4,0 Gew.-% Chitosan
   a3) 0,1 bis 15 Gew.-% filmbildendes Polymer;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 45 bis 98 Gew.-% mindestens eines polaren Lösungsmittels;
   a2) 0,1 bis 4,0 Gew.-% Chitosan
   a3) 0,1 bis 15 Gew.-% filmbildendes Polymer;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst.

Bevorzugt werden weiterhin kosmetische Produkte, umfassend
a) eine kosmetische Zubereitung, enthaltend bezogen auf ihr Gesamtgewicht
   a1) 45 bis 98 Gew.-% mindestens eines polaren Lösungsmittels;
   a2) 0,1 bis 4,0 Gew.-% Chitosan
   a3) 0,1 bis 15 Gew.-% filmbildendes Polymer;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Zusammenfassend ist ein besonders bevorzugter Gegenstand der vorliegenden Erfindung daher ein kosmetisches Produkt, umfassend
a) eine kosmetische Zubereitung, enthaltend bezogen auf ihr Gesamtgewicht
   a1) 45 bis 98 Gew.-% mindestens eines polaren Lösungsmittels;
   a2) 0,1 bis 4,0 Gew.-% Chitosan
   a3) 0,1 bis 15 Gew.-% filmbildendes Polymer;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Neben den beiden zuvor beschriebenen Bestandteilen a1), a2) und a3) können die erfindungsgemäßen kosmetischen Zubereitungen a) weitere Wirk- oder Hilfsstoffe enthalten, wobei insbesondere solche Wirk- oder Hilfsstoffe bevorzugt werden, welche die Herstellbarkeit, Applizierbarkeit und/oder kosmetische Wirkung erfindungsgemäßer kosmetischer Zubereitungen verbessern.

Zur Verbesserung der Herstellbarkeit, Applizierbarkeit und kosmetischen Wirkung enthält die kosmetische Zubereitung a) vorzugsweise nichtionisches Tensid a4), wobei besonders bevorzugte kosmetische Zubereitungen a) dadurch gekennzeichnet sind, dass sie bezogen auf ihr Gesamtgewicht 0,05 bis 4,0 Gew.-%, vorzugsweise 0,1 bis 2,0 Gew.-% und insbesondere 0,2 bis 1,0 Gew.-% nichtionisches Tensid a4) enthalten.

Bevorzugte nichtionische Tenside sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil, PEG-40 Hydrogenated Castor Oil oder PEG-60 Hydrogenated Castor Oil. Erfindungsgemäß besonders bevorzugt sind nichtionische Tenside ausgewählt aus der Gruppe der PEG-Derivate von hydriertem Ricinusöl, besonders bevorzugt aus der Gruppe PEG-40 Hydrogenated Castor Oil und PEG-60 Hydrogenated Castor Oil, insbesondere PEG-40 Hydrogenated Castor Oil enthält.

Ein weiterer bevorzugter Bestandteil erfindungsgemäßer kosmetischer Zubereitungen a) sind die kationischen Tenside a5). Bevorzugte kationische Tenside a5) sind ausgewählt unter quartären Ammoniumverbindungen, Esterquats und Amidoaminen. Die kationischen Tenside sind in der kosmetischen Zubereitung a), bezogen auf deren Gesamtgewicht, in Mengen von 0,01 bis 4,0 Gew.-%, vorzugsweise 0,05 bis 2,0 Gew.-%, und insbesondere 0,1 bis 1,0 Gew.-% enthalten. Kationsiche Tenside a5) aus der Gruppe der quartären Ammoniumverbindungen sind besonders bevorzugt.

Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Ganz besonders bevorzugte erfindungsgemäße kosmetische Zubereitungen a) sind dadurch gekennzeichnet, dass sie bezogen auf ihr Gesamtgewicht 0,01 bis 4,0 Gew.-%, vorzugsweise 0,05 bis 2,0 Gew.-%, und insbesondere 0,1 bis 1,0 Gew.-% (C₁₂ bis C₁₈)-Alkyltrimethylammoniumsalz(e) enthalten.

Als weitere geeignete Wirk- oder Hilfsstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Weitere Pflegestoffe sind Panthenol, Coffein, Nicotinamid und Sorbitol.

Als Pflegestoff können die erfindungsgemäßen Mittel weiterhin mindestens einen Pflanzenextrakt, aber auch Mono- bzw. Oligosaccharide und/oder Lipide enthalten.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

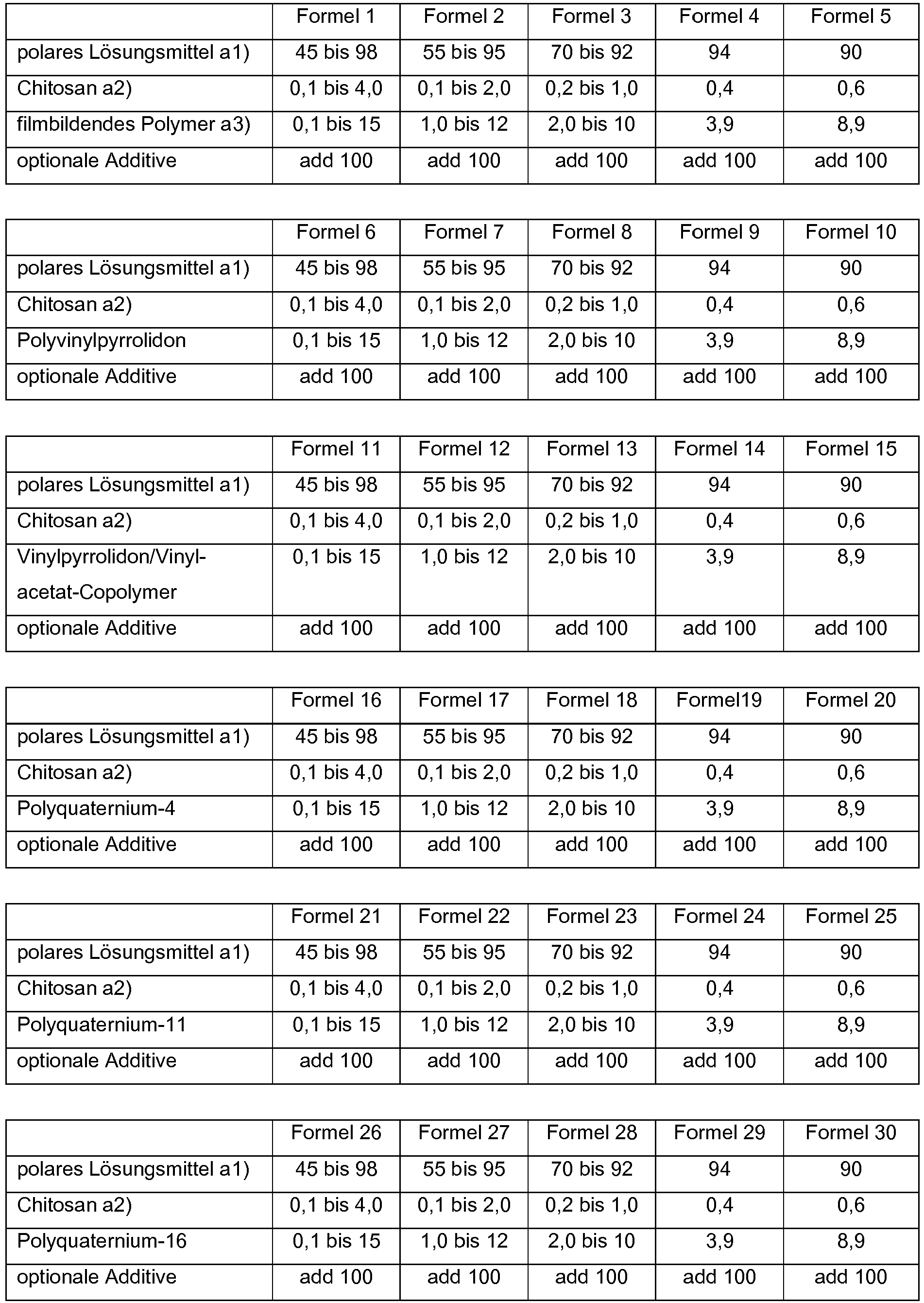

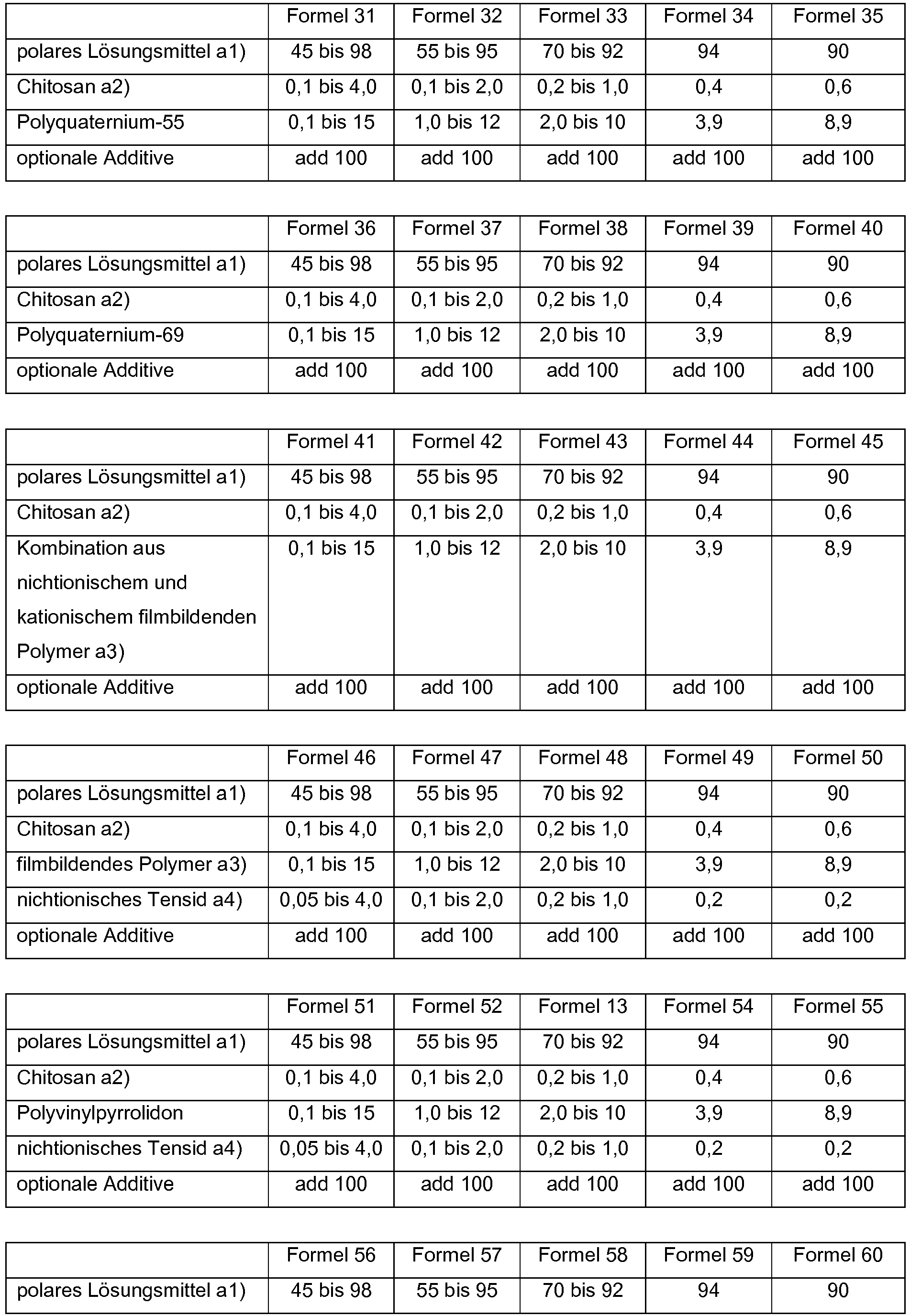

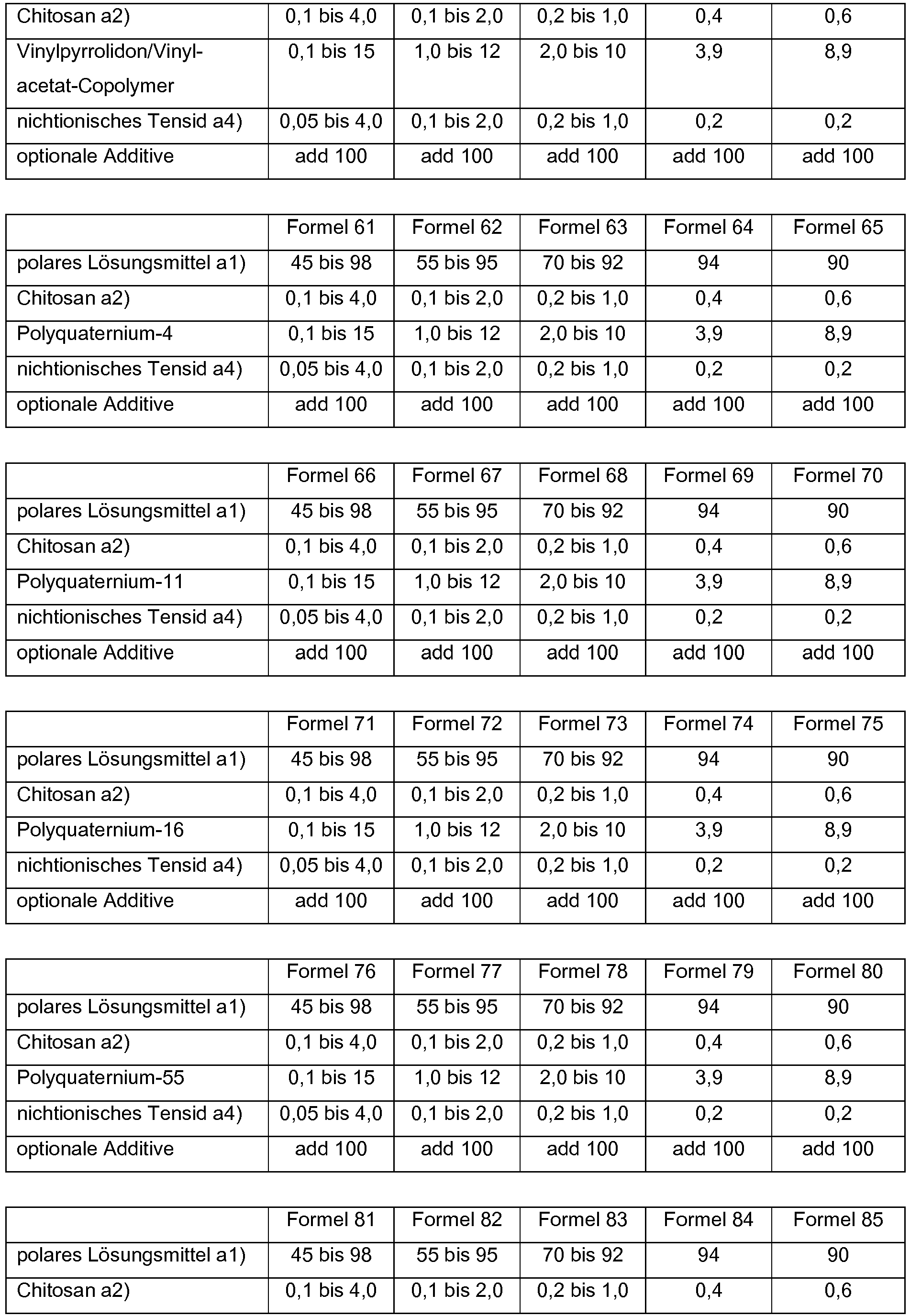

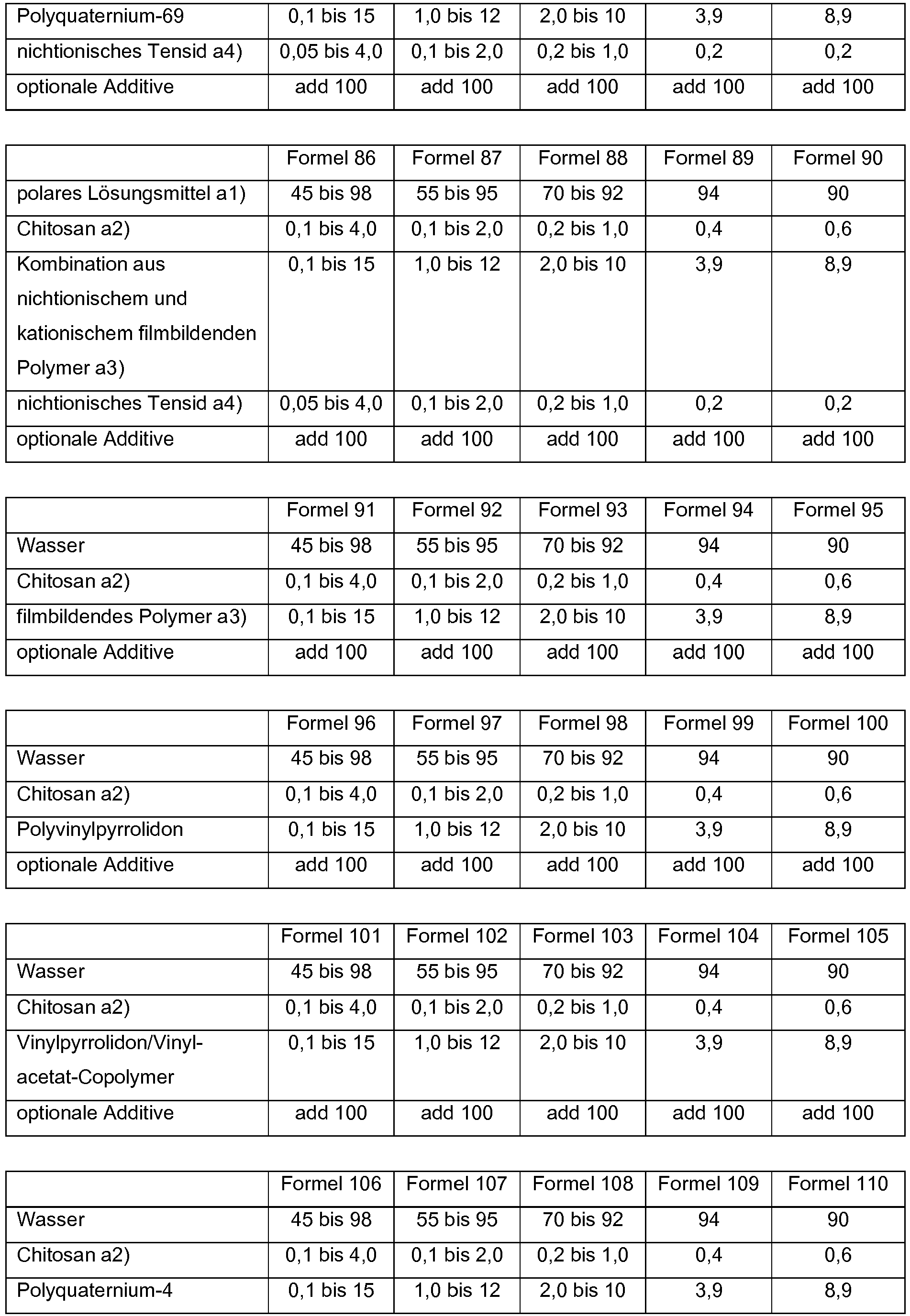

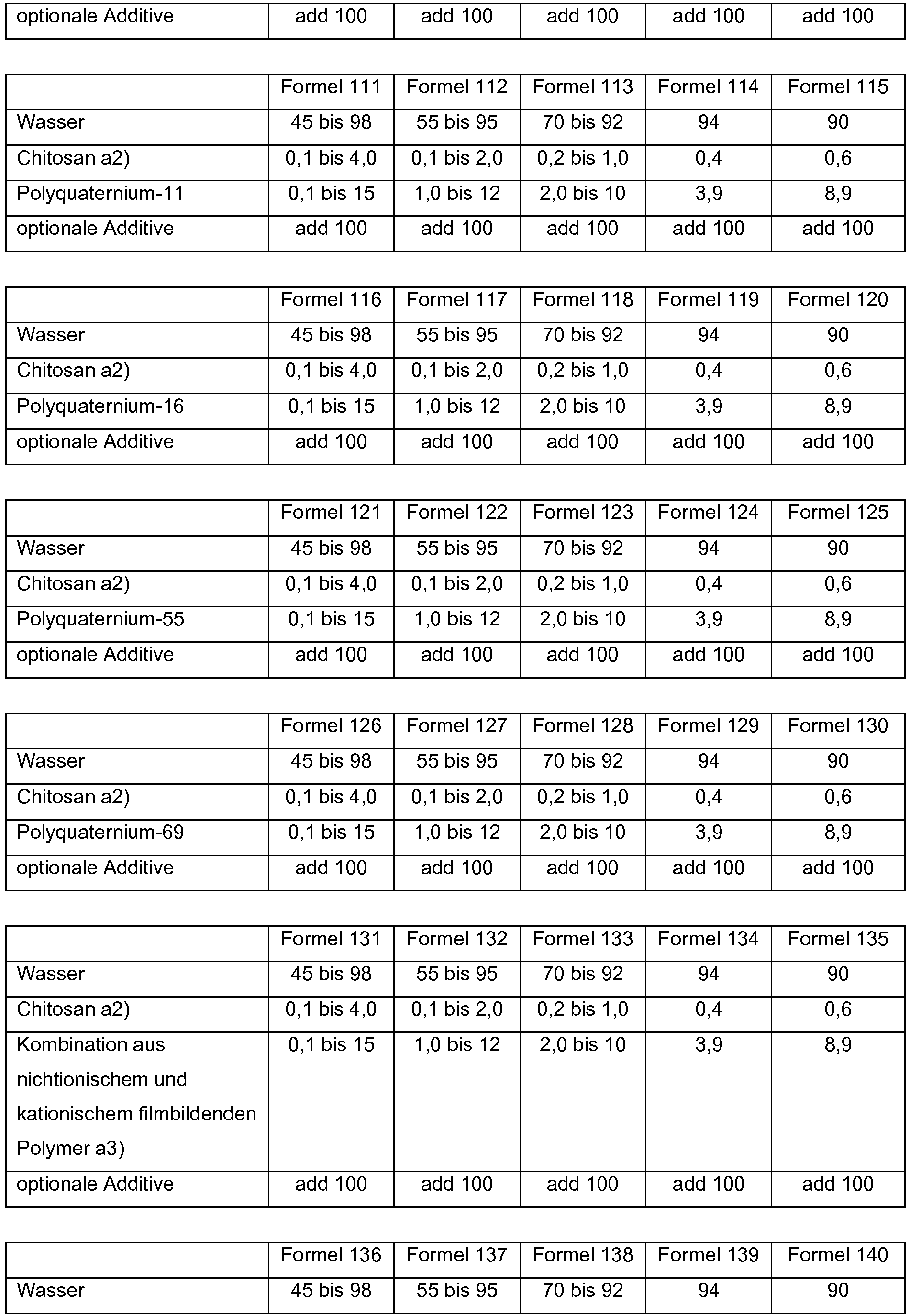

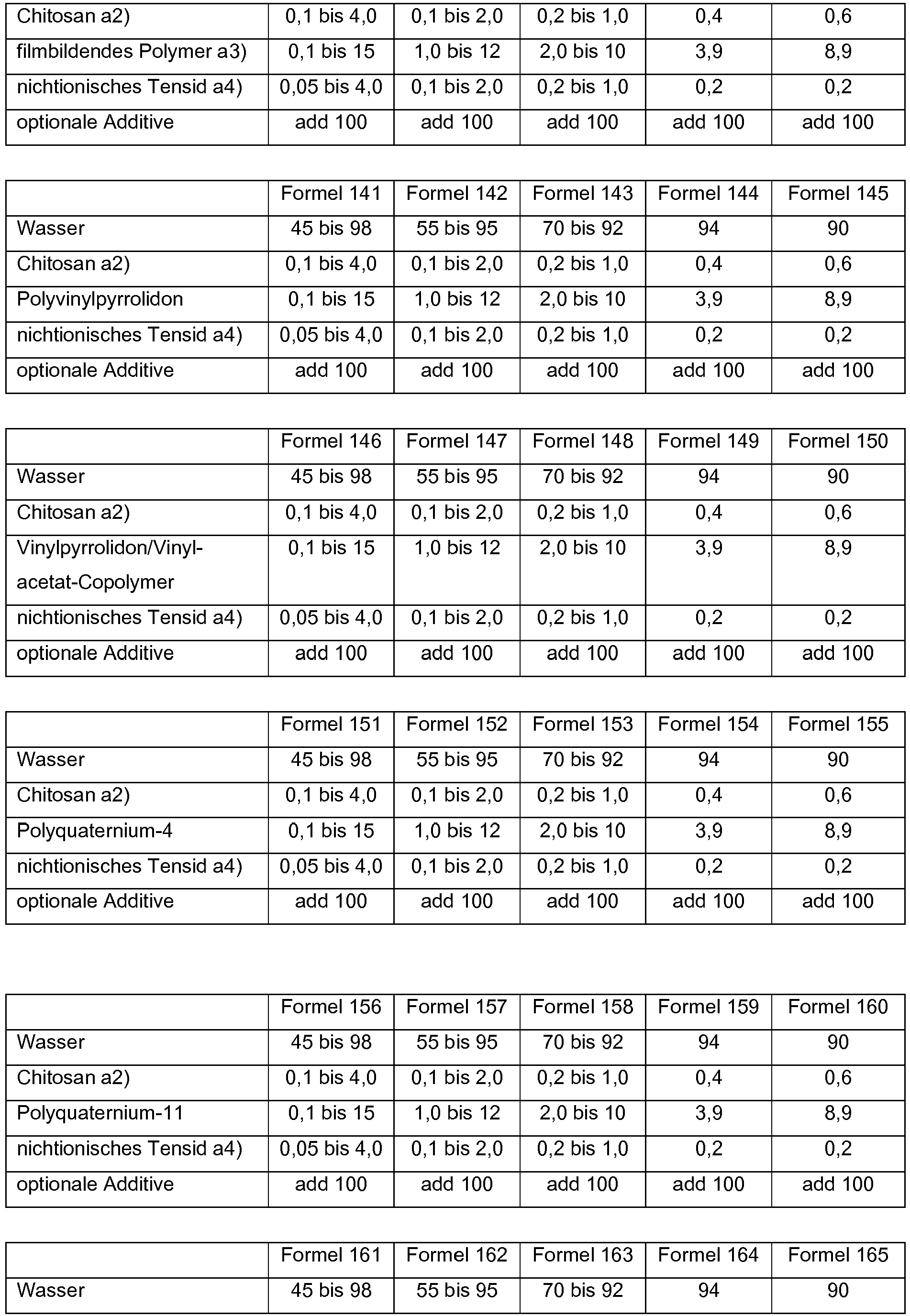

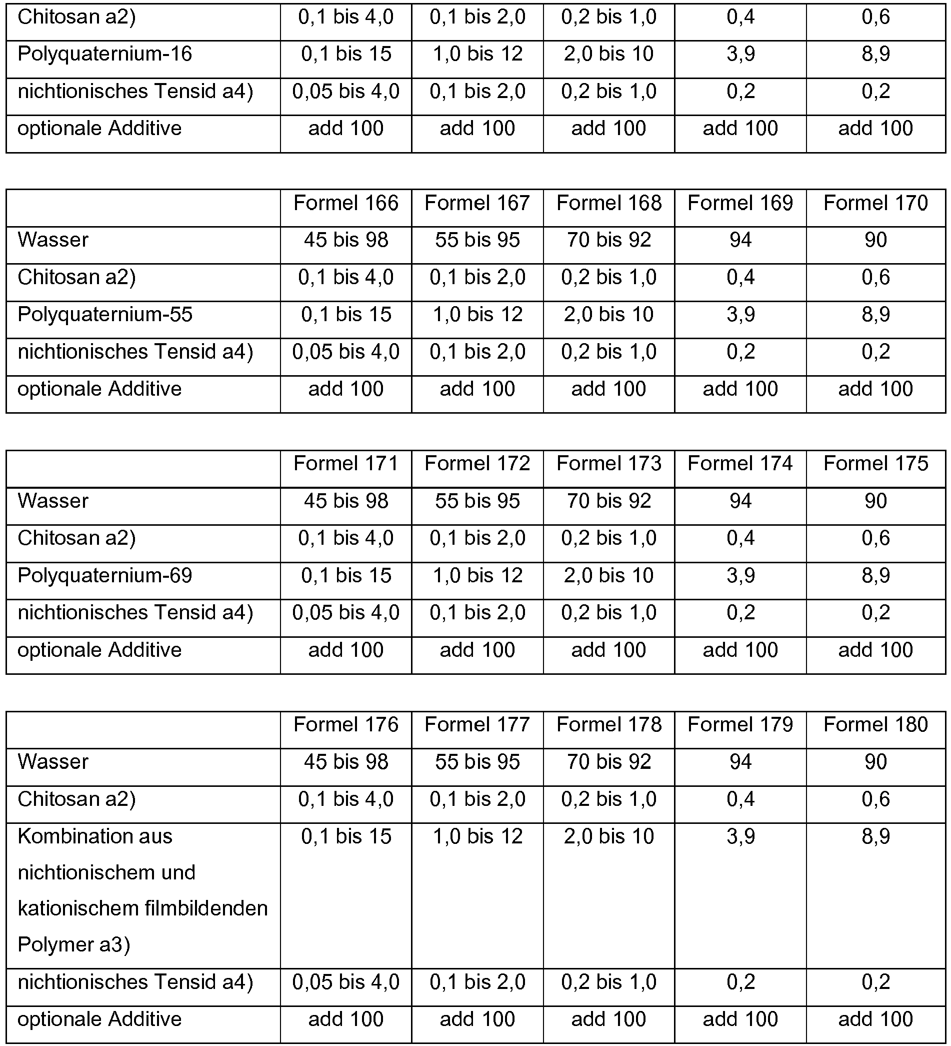

Ganz besonders bevorzugte kosmetische Zubereitungen enthalten neben den zuvor beschriebenen Bestandteilen a1) bis a5) nur geringe Mengen weiterer Wirk- und Hilfsstoffe. Kosmetische Zubereitungen, dadurch gekennzeichnet, dass der Gewichtsanteil der Bestandteile a1), a2) sowie, falls enthalten, a3) und/oder a4) und/oder a5) am Gesamtgewicht der kosmetischen Zubereitung mindestens 80 Gew.-%, vorzugsweise mindestens 90 Gew.-%, besonders bevorzugt mindestens 93 Gew.-% und insbesondere mindestens 97 Gew.-% beträgt, sind aufgrund ihrer einfachen Herstellbarkeit und guten kosmetischen Wirkung besonders bevorzugt. Ganz besonders bevorzugte kosmetische Zubereitungen bestehen, bezogen auf ihr Gesamtgewicht, zu mindestens 80 Gew.-%, vorzugsweise mindestens 87 Gew.-% und insbesondere mindestens 95 Gew.-% aus den Bestanteilen a1), a2) und a3).

Wie eingangs ausgeführt eignen sich die erfindungsgemäßen kosmetischen Zubereitungen a) in besonderer Weise für die Applikation mittels einer Vorrichtung zur Entspannungsverdampfung. Ein weiterer Gegenstand der vorliegenden Anmeldung ist daher die Verwendung einer kosmetischen Zubereitung a) enthaltend, bezogen auf ihr Gesamtgewicht,
a1) 45 bis 98 Gew.-% mindestens eines polaren Lösungsmittels;
a2) 0,1 bis 4,0 Gew.-% Chitosan
a3) 0,1 bis 15 Gew.-% filmbildendes Polymer
als Prozessgut in einer Vorrichtung zur Entspannungsverdampfung.

Gegenstand der vorliegenden Anmeldung ist zudem die Verwendung eines erfindungsgemäßen Produkts zur Beaufschlagung keratinhaltiger Fasern, insbesondere menschlicher Haare, mit einer kosmetischen Zubereitung a) bzw. zur temporären Verformung keratinhaltiger Fasern, insbesondere menschlicher Haare.

Ein Verfahren zur temporären Verformung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 45 bis 98 Gew.-% mindestens eines polaren Lösungsmittels;
a2) 0,1 bis 4,0 Gew.-% Chitosan;
a3) 0,1 bis 15 Gew.-% filmbildendes Polymer
beaufschlagt werden, ist ein weiterer Gegenstand der vorliegenden Anmeldung. Die kosmetische Zubereitung a) wird mittels der Vorrichtung zur Entspannungsverdampfung vorzugsweise in einen Sprühnebel überführt, welcher nachfolgend die keratinhaltigen Fasern beaufschlagt.

Um eine ausreichende Sprühwirkung zu erzielen, wird die kosmetische Zubereitung a) vorzugsweise auf Temperaturen oberhalb des Siedepunktes des in der kosmetischen Zubereitung a) enthaltenen polaren Lösungsmittels oder Lösungsmittelgemisches erhitzt.

Handelt es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches), wird die kosmetische Zubereitung vorzugsweise auf Temperaturen oberhalb 100°C, vorzugsweise auf Temperaturen von 100°C und 240°C, besonders bevorzugt auf Temperaturen von 140°C bis 160°C erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Ein bevorzugter Anmeldungsgegenstand ist ein Verfahren zur Farbveränderung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 45 bis 98 Gew.-% mindestens eines polaren Lösungsmittels;
a2) 0,1 bis 4,0 Gew.-% Chitosan;
a3) 0,1 bis 15 Gew.-% filmbildendes Polymer;
beaufschlagt werden, wobei
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter b1) überführt wird;
- nachfolgend der Zugang zwischen Vorratsbehälter und Behälter b1) durch ein Bauteil zur Durchflussregelung, mittels dessen der Durchfluss der kosmetischen Zubereitung a) aus dem Vorratsbehälter in den Behälter b1) unterbrochen werden kann, unterbrochen wird;
- nachfolgend die in dem gegen die Umgebung abgeschlossenen Behälter b1) befindliche kosmetische Zubereitung a) mittels einer Heizvorrichtung erhitzt wird, so dass der Druck im Inneren des Behälters b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung aus dem Behälter b1) in die Umgebung unter Minderung des zum Zeitpunkt der Behälteröffnung in dem Behälter b1) herrschenden Drucks, entspannt wird.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind besonders bevorzugt.

Bezüglich weiterer bevorzugter Ausführungsformen der erfindunsgemäßen Verwendungen und des erfindungsgemäßen Verfahrens gilt mutatis mutandis das zu den erfindungsgemäßen kosmetischen Zubereitungen a) und zu der Vorrichtung zur Entspannungsverdampfung b) Gesagte.

## Patentansprüche

1. Kosmetisches Produkt, umfassend
a) eine kosmetische Zubereitung, enthaltend bezogen auf ihr Gesamtgewicht
a1) 45 bis 98 Gew.-% mindestens eines polaren Lösungsmittels;
a2) 0,1 bis 4,0 Gew.-% Chitosan;
a3) 0,1 bis 15 Gew.-% filmbildendes Polymer;
b) eine Vorrichtung zur Entspannungsverdampfung der kosmetischen Zubereitung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung befüllten Innenraum des Behälters zu verschließen und zu öffnen,
b3) einer Heizvorrichtung, um die in dem geschlossenen Innenraum des Behälters befindliche kosmetische Zubereitung unter Druckerhöhung zu erhitzen, sowie die erhitzte kosmetische Zubereitung aus dem Innenraum des Behälters unter Druckminderung in die Umgebung zu entspannen,
b4) einer Düse, welche eine Zerstäubung der aus dem Behälter entweichenden kosmetischen Zubereitung ermöglicht,
c) einen Vorratsbehälter für die kosmetische Zubereitung, aus welchem die kosmetische Zubereitung in den Behälter gelangen kann, wobei
- der Zugang zwischen Vorratsbehälter und Behälter ein Bauteil zur Durchflussregelung aufweist, mittels dessen der Durchfluss der kosmetischen Zubereitung aus dem Vorratsbehälter in den Behälter unterbrochen werden kann,
- der Vorratsbehälter mindestens das Zehnfache Volumen, vorzugsweise mindestens das Zwanzigfache und insbesondere mindestens das Fünfzigfache Volumen des Behälters aufweist,
- der Druck im Inneren des Vorratsbehälters dem Umgebungsdruck entspricht.

2. Kosmetisches Produkt nach Anspruch 1, **dadurch gekennzeichnet, dass** der Gewichtsanteil des polaren Lösungsmittels a1) am Gesamtgewicht der kosmetischen Zubereitung 55 bis 95 Gew.-%, vorzugsweise 70 bis 92 Gew.-% beträgt.

3. Kosmetisches Produkt nach einem der vorherigen Ansprüche, **dadurch gekennzeichnet, dass** der Gewichtsanteil des Chitosans a2) am Gesamtgewicht der kosmetischen Zubereitung 0,1 bis 2,0 Gew.-%, vorzugsweise 0,2 bis 1,0 Gew.-% beträgt.

4. Kosmetisches Produkt nach einem der vorherigen Ansprüche, **dadurch gekennzeichnet, dass** die kosmetische Zubereitung bezogen auf ihr Gesamtgewicht 1,0 bis 12 Gew.-%, vorzugsweise 2,0 bis 10 Gew.-%, filmbildendes Polymer a3) enthält.

5. Kosmetisches Produkt nach Anspruch 4, **dadurch gekennzeichnet, dass** das filmbildende Polymer a3) ausgewählt ist aus der Gruppe der nichtionischen Polymere, vorzugsweise aus der Gruppe der Polyvinylpyrrolidone und Vinylpyrrolidon/Vinylacetat-Copolymere, vorzugsweise der Vinylpyrrolidon/Vinylacetat-Copolymere.

6. Kosmetisches Produkt nach Anspruch 4, **dadurch gekennzeichnet, dass** das filmbildende Polymer a3) ausgewählt ist aus der Gruppe der kationischen Polymere, vorzugsweise aus der Gruppe der kationischen Polymere mit den INCI Bezeichnungen Polyquaternium-4, Polyquaternium-11, Polyquaternium-16, Polyquaternium-55, Polyquaternium-69.

7. Verwendung einer kosmetischen Zubereitung a) enthaltend, bezogen auf ihr Gesamtgewicht,
a1) 45 bis 98 Gew.-% mindestens eines polaren Lösungsmittels;
a2) 0,1 bis 4,0 Gew.-% Chitosan;
a3) 0,1 bis 15 Gew.-% filmbildendes Polymer
als Prozessgut in einer Vorrichtung zur Entspannungsverdampfung der kosmetischen Zubereitung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung befüllten Innenraum des Behälters zu verschließen und zu öffnen,
b3) einer Heizvorrichtung, um die in dem geschlossenen Innenraum des Behälters befindliche kosmetische Zubereitung unter Druckerhöhung zu erhitzen, sowie die erhitzte kosmetische Zubereitung aus dem Innenraum des Behälters unter Druckminderung in die Umgebung zu entspannen,
b4) einer Düse, welche eine Zerstäubung der aus dem Behälter entweichenden kosmetischen Zubereitung ermöglicht,
c) einem Vorratsbehälter für die kosmetische Zubereitung, aus welchem die kosmetische Zubereitung in den Behälter gelangen kann, wobei
- der Zugang zwischen Vorratsbehälter und Behälter ein Bauteil zur Durchflussregelung aufweist, mittels dessen der Durchfluss der kosmetischen Zubereitung aus dem Vorratsbehälter in den Behälter unterbrochen werden kann,
- der Vorratsbehälter mindestens das Zehnfache Volumen, vorzugsweise mindestens das Zwanzigfache und insbesondere mindestens das Fünfzigfache Volumen des Behälters aufweist,
- der Druck im Inneren des Vorratsbehälters dem Umgebungsdruck entspricht.

8. Verwendung eines Produkts nach einem der Ansprüche 1 bis 6 zur Beaufschlagung keratinhaltiger Fasern, insbesondere menschlicher Haare, mit einer kosmetischen Zubereitung a).

9. Verwendung eines Produkts nach einem der Ansprüche 1 bis 6 zur temporären Verformung keratinhaltiger Fasern, insbesondere menschlicher Haare.

10. Verfahren zur temporären Verformung keratinhaltiger Fasern, insbesondere menschlicher Haare, bei welchem die keratinhaltigen Fasern mittels einer Vorrichtung zur Entspannungsverdampfung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
c) einem Vorratsbehälter für die kosmetische Zubereitung, aus welchem die kosmetische Zubereitung in den Behälter gelangen kann, wobei
- der Zugang zwischen Vorratsbehälter und Behälter ein Bauteil zur Durchflussregelung aufweist, mittels dessen der Durchfluss der kosmetischen Zubereitung aus dem Vorratsbehälter in den Behälter unterbrochen werden kann,
- der Vorratsbehälter mindestens das Zehnfache Volumen, vorzugsweise mindestens das Zwanzigfache und insbesondere mindestens das Fünfzigfache Volumen des Behälters aufweist,
- der Druck im Inneren des Vorratsbehälters dem Umgebungsdruck entspricht,
mit einer kosmetischen Zubereitung a) enthaltend bezogen auf ihr Gesamtgewicht
a1) 45 bis 98 Gew.-% mindestens eines polaren Lösungsmittels;
a2) 0,1 bis 4,0 Gew.-% Chitosan;
a3) 0,1 bis 15 Gew.-% filmbildendes Polymer
beaufschlagt werden.

11. Verfahren nach Anspruch 10, **dadurch gekennzeichnet, dass**
- aus dem Vorratsbehälter eine Teilmenge der in diesem Vorratsbehälter befindlichen kosmetischen Zubereitung in den Behälter überführt wird;
- nachfolgend der Zugang zwischen Vorratsbehälter und Behälter durch ein Bauteil zur Durchflussregelung, mittels dessen der Durchfluss der kosmetischen Zubereitung aus dem Vorratsbehälter in den Behälter unterbrochen werden kann, unterbrochen wird;
- nachfolgend die in dem gegen die Umgebung abgeschlossenen Behälter befindliche kosmetische Zubereitung mittels einer Heizvorrichtung erhitzt wird, so dass der Druck im Inneren des Behälters auf Werte oberhalb des Umgebungsdrucks, vorzugsweise auf Werte zwischen 1,1 und 8 bar, insbesondere auf werte zwischen 1,2 und 4 bar ansteigt;
- nachfolgend der unter einem Druck oberhalb des Umgebungsdrucks stehende Behälter in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung aus dem Behälter in die Umgebung unter Minderung des zum Zeitpunkt der Behälteröffnung in dem Behälter herrschenden Drucks, entspannt wird.

## Claims

1. A cosmetic product comprising
a) a cosmetic preparation containing, based on the total weight thereof,
a1) 45 to 98 wt.% of at least one polar solvent;
a2) 0.1 to 4.0 wt.% chitosan;
a3) 0.1 to 15 wt.% film-forming polymer;
b) a device for flash evaporation of the cosmetic preparation, comprising
b1) a container defining a closed interior in which the cosmetic preparation can be received,
b2) a valve or a similarly functioning closure element for closing and opening the interior of the container that is at least partially filled with the cosmetic preparation,
b3) a heating device for heating the cosmetic preparation located in the closed interior of the container under increased pressure, and for releasing, under reduced pressure, the heated cosmetic preparation from the interior of the container into the surroundings,
b4) a nozzle which allows atomization of the cosmetic preparation escaping from the container,
c) a reservoir for the cosmetic preparation, from which the cosmetic preparation can enter the container, wherein
- the access between the reservoir and the container has a component for controlling flow, by means of which the flow of the cosmetic preparation from the reservoir into the container can be interrupted,
- the reservoir has a volume which is at least ten times, preferably at least twenty times and in particular at least fifty times the volume of the container,
- the pressure inside the reservoir corresponds to the ambient pressure.

2. The cosmetic product according to claim 1, **characterized in that** the proportion by weight of the polar solvent a1) with respect to the total weight of the cosmetic preparation is 55 to 95 wt.%, preferably 70 to 92 wt.%.

3. The cosmetic product according to one of the preceding claims, **characterized in that** the proportion by weight of the chitosan a2) with respect to the total weight of the cosmetic preparation is 0.1 to 2.0 wt.%, preferably 0.2 to 1.0 wt.%.

4. The cosmetic product according to one of the preceding claims, **characterized in that** the cosmetic preparation contains, based on the total weight thereof, 1.0 to 12 wt.%, preferably 2.0 to 10 wt.% film-forming polymer a3).

5. The cosmetic product according to claim 4, **characterized in that** the film-forming polymer a3) is selected from the group of nonionic polymers, preferably from the group of polyvinylpyrrolidones and vinylpyrrolidone/vinyl acetate copolymers, preferably of vinylpyrrolidone/vinyl acetate copolymers.

6. The cosmetic product according to claim 4, **characterized in that** the film-forming polymer a3) is selected from the group of cationic polymers, preferably from the group of cationic polymers having the INCI names polyquaternium-4, polyquaternium-11, polyquaternium-16, polyquaternium-55 and polyquaternium-69.

7. The use of a cosmetic preparation a) containing, based on the total weight thereof,
a1) 45 to 98 wt.% of at least one polar solvent;
a2) 0.1 to 4.0 wt.% chitosan;
a3) 0.1 to 15 wt.% film-forming polymer
as a process material in a device for flash evaporation of the cosmetic preparation, comprising
b1) a container defining a closed interior in which the cosmetic preparation can be received,
b2) a valve or a similarly functioning closure element for closing and opening the interior of the container that is at least partially filled with the cosmetic preparation,
b3) a heating device for heating the cosmetic preparation located in the closed interior of the container under increased pressure, and for releasing, under reduced pressure, the heated cosmetic preparation from the interior of the container into the surroundings,
b4) a nozzle which allows atomization of the cosmetic preparation escaping from the container,
c) a reservoir for the cosmetic preparation, from which the cosmetic preparation can enter the container, wherein
- the access between the reservoir and the container has a component for controlling flow, by means of which the flow of the cosmetic preparation from the reservoir into the container can be interrupted,
- the reservoir has a volume which is at least ten times, preferably at least twenty times and in particular at least fifty times the volume of the container,
- the pressure inside the reservoir corresponds to the ambient pressure.

8. The use of a product according to one of claims 1 to 6 for applying a cosmetic preparation a) to keratin-containing fibers, in particular human hair.

9. The use of a product according to one of claims 1 to 6 for temporarily shaping keratin-containing fibers, in particular human hair.

10. A method for temporarily shaping keratin-containing fibers, in particular human hair, in which, by means of a device for flash evaporation comprising
b1) a container defining a closed interior in which the cosmetic preparation can be received,
b2) a valve or a similarly functioning closure element for closing and opening the interior of the container that is at least partly filled with the cosmetic preparation a),
b3) a heating device for heating, under increased pressure, the cosmetic preparation a) which is located in the closed interior of the container, and releasing, under reduced pressure, the heated cosmetic preparation a) from the interior of the container into the surroundings,
b4) a nozzle which allows atomization of the cosmetic preparation a) escaping from the container,
c) a reservoir for the cosmetic preparation, from which the cosmetic preparation can enter the container, wherein
- the access between the reservoir and the container has a component for controlling flow, by means of which the flow of the cosmetic preparation from the reservoir into the container can be interrupted,
- the reservoir has a volume which is at least ten times, preferably at least twenty times and in particular at least fifty times the volume of the container,
- the pressure inside the reservoir corresponds to the ambient pressure,
a cosmetic preparation a) containing, based on the total weight thereof,
a1) 45 to 98 wt.% of at least one polar solvent;
a2) 0.1 to 4.0 wt.% chitosan;
a3) 0.1 to 15 wt.% film-forming polymer is applied to the keratin-containing fibers.

11. The method according to claim 10, **characterized in that**
- some of the cosmetic preparation located in the reservoir is transferred from said reservoir into the container;
- using a component for controlling flow, by means of which the flow of the cosmetic preparation from the reservoir into the container can be interrupted, the access between the reservoir and the container is subsequently interrupted;
- the cosmetic preparation located in the container which is sealed against the surroundings is subsequently heated by means of a heating device such that the pressure inside the container increases to values above the ambient pressure, preferably to values between 1.1 and 8 bar, in particular to values between 1.2 and 4 bar;
- the container which is pressurized above the ambient pressure is subsequently opened in such a manner that the discharge of at least some, preferably at least 50 wt.%, more preferably at least 80 wt.% and in particular at least 90 wt.%, of the cosmetic preparation located in the container b1) is released from the container into the surroundings by the pressure prevailing in the container at the time at which the container is opened being reduced.

## Revendications

1. Produit cosmétique, comprenant
a) une préparation cosmétique contenant, par rapport à son poids total,
a1) 45 à 98 % en poids d'au moins un solvant polaire ;
a2) 0,1 à 4,0 % en poids de chitosane ;
a3) 0,1 à 15 % en poids d'un polymère filmogène ;
b) un dispositif d'évaporation par détente de la préparation cosmétique, comportant
b1) un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de la préparation cosmétique,
b3) un dispositif de chauffage permettant de chauffer la préparation cosmétique présente dans l'espace intérieur fermé du récipient tout en augmentant la pression, et de détendre la préparation cosmétique chauffée hors de l'espace intérieur du récipient tout en réduisant la pression dans l'environnement,
b4) une buse permettant d'atomiser la préparation cosmétique s'échappant du récipient,
c) un récipient de stockage destiné à la préparation cosmétique, à partir duquel la préparation cosmétique peut pénétrer dans le récipient,
- l'accès entre le récipient de stockage et le récipient présentant un élément de régulation de flux, au moyen duquel le flux de la préparation cosmétique depuis le récipient de stockage jusqu'au récipient peut être interrompu ;
- le récipient de stockage présentant au moins dix fois, de préférence au moins vingt fois et en particulier au moins cinquante fois le volume du récipient,
- la pression à l'intérieur du récipient de stockage correspondant à la pression ambiante.

2. Produit cosmétique selon la revendication 1, **caractérisé en ce que** le pourcentage en poids du solvant polaire a1) par rapport au poids total de la préparation cosmétique est de 55 à 95 % en poids, de préférence de 70 à 92 % en poids.

3. Produit cosmétique selon l'une des revendications précédentes, **caractérisé en ce que** le pourcentage en poids du chitosane a2) par rapport au poids total de la préparation cosmétique est de 0,1 à 2,0 % en poids, de préférence de 0,2 à 1,0 % en poids.

4. Produit cosmétique selon l'une des revendications précédentes, **caractérisé en ce que** la préparation cosmétique contient, par rapport à son poids total, 1,0 à 12 % en poids, de préférence 2,0 à 10 % en poids, d'un polymère filmogène a3).

5. Produit cosmétique selon la revendication 4, **caractérisé en ce que** le polymère filmogène a3) est choisi dans le groupe des polymères non ioniques, de préférence dans le groupe des polyvinylpyrrolidones et des copolymères vinylpyrrolidone/acétate de vinyle, de préférence des copolymères vinylpyrrolidone/acétate de vinyle.

6. Produit cosmétique selon la revendication 4, **caractérisé en ce que** le polymère filmogène a3) est choisi dans le groupe des polymères cationiques, de préférence dans le groupe des polymères cationiques portant les dénominations INCI Polyquaternium-4, Polyquaternium-11, Polyquaternium-16, Polyquaternium-55, Polyquaternium-69.

7. Utilisation d'une préparation cosmétique a) contenant, par rapport à son poids total,
a1) 45 à 98 % en poids d'au moins un solvant polaire ;
a2) 0,1 à 4,0 % en poids de chitosane ;
a3) 0,1 à 15 % en poids d'un polymère filmogène
en tant que produit de traitement dans un dispositif d'évaporation par détente de la préparation cosmétique, le dispositif d'évaporation comportant
b1) un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de la préparation cosmétique,
b3) un dispositif de chauffage permettant de chauffer la préparation cosmétique présente dans l'espace intérieur fermé du récipient tout en augmentant la pression, et de détendre la préparation cosmétique chauffée hors de l'espace intérieur du récipient tout en réduisant la pression dans l'environnement,
b4) une buse permettant d'atomiser la préparation cosmétique s'échappant du récipient,
c) un récipient de stockage destiné à la préparation cosmétique, à partir duquel la préparation cosmétique peut pénétrer dans le récipient,
- l'accès entre le récipient de stockage et le récipient présentant un élément de régulation de flux, au moyen duquel le flux de la préparation cosmétique depuis le récipient de stockage jusqu'au récipient peut être interrompu ;
- le récipient de stockage présentant au moins dix fois, de préférence au moins vingt fois et en particulier au moins cinquante fois le volume du récipient,
- la pression à l'intérieur du récipient de stockage correspondant à la pression ambiante.

8. Utilisation d'un produit selon l'une des revendications 1 à 6 pour l'application d'une préparation cosmétique a) sur des fibres kératiniques, en particulier sur des cheveux humains.

9. Utilisation d'un produit selon l'une des revendications 1 à 6 pour la déformation temporaire de fibres kératiniques, en particulier de cheveux humains.

10. Procédé pour déformer temporairement des fibres kératiniques, en particulier des cheveux humains, selon lequel on applique sur les fibres kératiniques, au moyen d'un dispositif d'évaporation par détente comportant
b1) un récipient définissant un espace intérieur fermé dans lequel la préparation cosmétique peut être reçue,
b2) une soupape ou un élément de fermeture à action comparable permettant de fermer et d'ouvrir l'espace interne du récipient rempli au moins partiellement de la préparation cosmétique a),
b3) un dispositif de chauffage permettant de chauffer la préparation cosmétique a) présente dans l'espace intérieur fermé du récipient tout en augmentant la pression, et de détendre la préparation cosmétique a) chauffée hors de l'espace intérieur du récipient tout en réduisant la pression dans l'environnement,
b4) une buse permettant d'atomiser la préparation cosmétique a) s'échappant du récipient,
c) un récipient de stockage destiné à la préparation cosmétique, à partir duquel la préparation cosmétique peut pénétrer dans le récipient,
- l'accès entre le récipient de stockage et le récipient présentant un élément de régulation de flux, au moyen duquel le flux de la préparation cosmétique depuis le récipient de stockage jusqu'au récipient peut être interrompu ;
- le récipient de stockage présentant au moins dix fois, de préférence au moins vingt fois et en particulier au moins cinquante fois le volume du récipient,
- la pression à l'intérieur du récipient de stockage correspondant à la pression ambiante,
une préparation cosmétique a) contenant, par rapport à son poids total,
a1) 45 à 98 % en poids d'au moins un solvant polaire ;
a2) 0,1 à 4,0 % en poids de chitosane ;
a3) 0,1 à 15 % en poids d'un polymère filmogène.

11. Procédé selon la revendication 10, **caractérisé en ce**
- **qu'**une fraction de la préparation cosmétique présente dans le récipient de stockage est transférée de ce récipient de stockage au récipient ;
- **qu'**on interrompt ensuite l'accès entre le récipient de stockage et le récipient par un élément de régulation de flux, au moyen duquel le flux de la préparation cosmétique depuis le récipient de stockage jusqu'au récipient peut être interrompu ;
- **qu'**on chauffe ensuite la préparation cosmétique présente dans le récipient isolé de l'environnement au moyen d'un dispositif de chauffage, de sorte que la pression à l'intérieur du récipient dépasse la pression ambiante et atteint de préférence des valeurs comprises entre 1,1 et 8 bar, en particulier entre 1,2 et 4 bar ;
- **que** le récipient se trouvant à une pression supérieure à la pression ambiante est ensuite ouvert de manière à détendre au moins une fraction, de préférence au moins 50 % en poids, de façon préférée au moins 80 % en poids et en particulier au moins 90 % en poids de la préparation cosmétique présente dans le récipient b1) et à provoquer sa sortie dans l'environnement tout en réduisant la pression régnant dans le récipient au moment de l'ouverture de celui-ci.
